# EUROPEAN PATENT SPECIFICATION

(11) **EP 3 893 242 B1**
(45) Date of publication and mention of the grant of the patent: **11.03.2026**
(21) Application number: 19888541.0
(22) Date of filing: 20.08.2019
(51) Int. Cl.: G16B 20/40, G16B 25/00, G01N 33/68, C12Q 1/6883, G16B 25/10

(54) **NOVEL TARGET PROTEIN, AND COMPANION DIAGNOSIS BIOMARKER DISCOVERY SYSTEM AND METHOD THEREFOR**
NEUARTIGES ZIELPROTEIN UND SYSTEM ZUR ENTDECKUNG VON BEGLEITDIAGNOSEBIOMARKERN SOWIE VERFAHREN DAFÜR
NOUVELLE PROTÉINE CIBLE ET SYSTÈME DE DÉCOUVERTE DE BIOMARQUEURS DE DIAGNOSTIC COMPAGNON ET PROCÉDÉ ASSOCIÉ

(30) Priority: 30.11.2018 KR 20180151779
(43) Date of publication of application: 13.10.2021
(73) Proprietor: 3Billion, Seongdong-Gu Seoul 04766 (KR)
(72) Inventor: LEE, Jung Sul, Yongin-Si Gyeonggi-do 17115 (KR)
(74) Representative: Grünecker Patent- und Rechtsanwälte PartG mbB
(86) International application number: PCT/KR2019/010563
(87) International publication number: WO 2020/111451

(56) References cited:
- JP-A- 2005 270 093
- KR-A- 20120 079 295
- KR-A- 20160 086 145
- KR-A- 20160 086 145
- KR-A- 20170 032 892
- KR-A- 20170 047 037
- KR-A- 20180 038 346
- KR-A- 20180 038 346

## Description

### TECHNICAL FIELD

The present disclosure relates to a system and method for discovering a novel target protein and a companion diagnostic biomarker therefor using prognostic data.

### BACKGROUND ART

Precision diagnostic medical science is a concept that precisely presents a treatment method of a patient according to the characteristics of the patient.

Classically, a patient group is divided into a small number of subgroups, such as gender and age groups, to search for differences in treatment effects. If treatment effects differ according to these characteristics, patients have been subgrouped according to such characteristics, and then suitable treatment methods have been presented. However, as it becomes technically possible to obtain a large amount of individual genetic information, efforts are being made to present a treatment method that can be expected to have the maximal treatment effect in a small number of patient groups who share the same or similar genetic characteristics. Accordingly, it is an important issue to search for and discover genetic features for selecting a patient group that can be expected to have the maximal treatment effect.

Recently, as a technology that can measure the genetic features of cancer cells in large quantities has been developed, attempts are underway to subgroup a patient group using such a large quantity of characteristics by measuring characteristic values such as 1) gene expression level, 2) degree of gene promoter methylation, and 3) gene copy number variation simultaneously for tens of thousands of genes.

However, traditional grouping operations are performed using various clustering techniques and are somewhat mechanically grouped. Thus, there is an inconvenience in that the biological features shared by the subgroups found in this manner needs to be found again. If an attempt is made to subgroup a patient group with a characteristic value having an excessively large volume, it becomes unclear which characteristic value is important for subgrouping of the patient group.

Patent document KR20160086145A (NAT UNIV SUNCHON IACF [KR]) discloses a method of selecting genes for predicting breast cancer prognosis, which includes classifying patients into a high expression group and a low expression group. Patent document KR20170032892A (INDUSTRY-ACADEMIC COOP FOUND OF SUNCHON NAT UNIV [KR]) discloses a method for predicting the prognosis of ovarian cancer by means of classifying patients into high- or low-expression clusters according to gene expression data. Finally, patent document KR20180038346A (GWANGJU INST SCIENCE & TECH [KR]) relates to a method for predicting the prognosis of breast cancer through gene expression data.

### DISCLOSURE OF INVENTION

### TECHNICAL PROBLEM

A technical task of the present disclosure is to provide a system and method for discovering a novel target protein effective in treating a patient group and a biomarker for dividing the patient group that is expected to have similar therapeutic responsiveness.

### SOLUTION TO PROBLEM

The present invention is defined in the independent claims. The dependent claims define embodiments of the present invention.

The system for discovering a novel target protein and a companion diagnostic biomarker therefor according to an embodiment of the present disclosure comprises: a patient group classification unit specifying a single gene and dividing a high-expression patient group and a low-expression patient group according to an expression level of the single gene; a prognostic association calculation unit calculating prognostic association values of all genes in the high-expression patient group and the low-expression patient group; a prognostic association comparison unit comparing prognostic association values of the high-expression patient group and prognostic association values of the low-expression patient group for all genes; and a biomarker selection unit selecting, from the comparison value, the single gene to which the patient group is initially divided as a biomarker to divide the patient group.

A novel target protein selection unit selecting a novel target protein as the prognostic association value in a high-expression patient group or a low-expression patient group based on the biomarker may be further included.

The prognostic association value may be calculated based on a gene expression level, disease recurrence period, and whether a disease has recurred or not, which are prognostic data of a patient.

A reference expression value for dividing the high-expression patient group and the low-expression patient group may be performed by an average value of a patient group or a step-miner algorithm.

The prognostic association value may be generated by any one of a log-rank test, a Cox hazard ratio, or a log-rank test based on iterative patient partitioning method.

A comparison of the prognostic association value of the high-expression patient group and the prognostic association value of the low-expression patient group may be performed by any one of a Pearson's correlation coefficient, a Euclidean distance, a Mahalanobis distance, or a Tanimoto coefficient.

In addition to the technical tasks of the present disclosure mentioned above, other features and advantages of the present disclosure will be described below or will be clearly understood by those having ordinary skill in the technical field to which the present disclosure pertains from such technology and description.

### ADVANTAGEOUS EFFECTS OF INVENTION

According to the present disclosure as described above, there are the following effects.

The present disclosure allows the simultaneously discovery of an effective target protein in a patient group that shares genetic features similar to a biomarker for predicting responsiveness to treatment.

The present disclosure allows the discovery of a novel target protein and a companion diagnostic biomarker therefor without prescription data for a drug inhibiting a protein that is expected to be a novel target.

In addition, other features and advantages of the present disclosure may be newly recognized through exemplary embodiments of the present disclosure.

### BRIEF DESCRIPTION OF DRAWINGS

FIG. 1 is a block diagram of a system for discovering a novel target protein and a biomarker therefor according to an embodiment of the present disclosure;
FIG. 2 is a diagram illustrating a portion of the actual results calculated using a system for discovering a novel target protein and a companion diagnostic biomarker therefor according to an embodiment of the present disclosure;
FIG. 3 is an experimental diagram illustrating the prognostic association values of PAK1 in a low-expression group of NFKBIE;
FIG. 4 is an experimental diagram illustrating the prognostic association values of PAK1 in a high-expression group of NFKBIE;
FIG. 5 is an experimental diagram illustrating the prognostic association values of PAK1 in all patients; and
FIG. 6 is a flowchart of a method for discovering a novel target protein and a companion diagnostic biomarker therefor according to an embodiment of the present disclosure.

### DESCRIPTION OF EMBODIMENTS

In the specification, in adding reference numerals for elements in each drawing, it should be noted that like reference numerals designate like elements wherever possible even though elements are shown in other drawings.

The terms described in the present specification should be understood as follows.

It will be understood that the terms "comprise" or "have" do not preclude the presence or addition of one or more other features, integers, steps, operations, elements, components, or combinations thereof.

Hereinafter, preferred embodiments of the present disclosure will be described in detail with reference to the accompanying drawings.

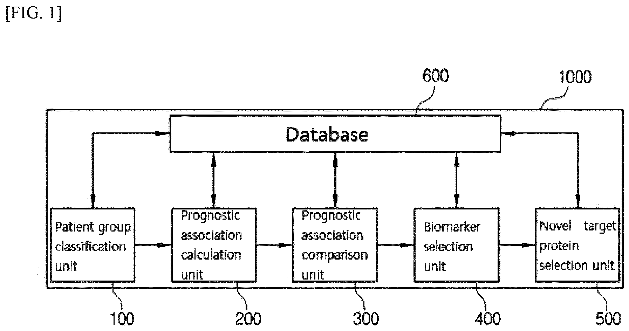

FIG. 1 is a block diagram of a system for discovering a novel target protein and a biomarker therefor according to an embodiment of the present disclosure.

Referring to FIG. 1, a system 1000 for discovering a novel target protein and a companion diagnostic biomarker therefor according to an embodiment of the present disclosure includes a patient group classification unit 100, a prognostic association calculation unit 200, a prognostic association comparison unit 300, a biomarker selection unit 400, a novel target protein selection unit 500, and a database 600.

The database 600 may store prognostic data of a patient. Here, the prognostic data is information regarding the gene expression level for a total of 20,000 genes for patients, whether patients have died (or a disease has recurred), and the time period until patients will die (or recurrence period).

After specifying an arbitrary single gene using the prognostic data, the patient group classification unit 100 may divide a high-expression patient group and a low-expression patient group according to a specific expression level of the single gene.

The reference expression value for dividing the high-expression patient group and the low-expression patient group is an average value of the patient group, which is a commonly and widely used method, or can be found using a stepminer algorithm that can statistically find out where the change in value is noticeable when it is in the form of a single calculation function (step function) when the expression values of two patient groups are sorted in ascending order based on the expression level (stepminer document: Extracting binary signals from microarray time-course data). In an embodiment of the present disclosure, the reference expression value for dividing the high-expression patient group and the low-expression patient group will be described by limiting the use of the average value of the patient group or the stepminer algorithm, but all conventional ones used to find the reference expression value in the pertinent field may be used.

The prognostic association calculation unit 200 may calculate prognostic association values for all genes in each of the high-expression patient group and the low-expression patient group.

The prognostic association value may be calculated by the gene expression level, the duration of disease recurrence, and whether the disease recurs, which are prognostic data of a patient.

The prognostic association value may be generated by any one of a log-rank test, a Cox hazard ratio, or a log-rank test based on iterative patient partitioning method, which are commonly used.

The log-rank test or the Cox hazard ratio is a technique known to those skilled in the art, and thus a detailed description thereof will be omitted.

For the contents on the log-rank test based on iterative patient partitioning method, Robust method for identification of prognostic gene signatures from gene expression profiles, Woogwang Sim, Jungsul Lee & Chulhee Choi, Scientific Reports, volume 7, Article number: 16926 (2017) may be referred.

The prognostic association comparison unit 300 may compare the prognostic association value in the high-expression patient group and the prognostic association value in the low-expression patient group for all genes.

For example, for all gene numbers 1 to N, the patient group classification unit 100 specifies gene m, and divides patients into a high-expression patient group (mH) and a low-expression patient group (mL) according to the expression level of gene m.

In addition, the prognostic association calculation unit 200 calculates prognostic associations of all genes from gene numbers 1 to N.

In addition, the prognostic association comparison unit 300 compares mLi and mHi values for all genes i (numbers 1 to N). mL1 is the prognostic association value of gene 1 in the group of patients with low expression of gene m (mL).

In other words, the prognostic association comparison unit 300 determines whether the comparison of the mLi and mHi values is sufficiently large, and the degree of similarity between the mLi and mHi values may be performed by any one of a Pearson's correlation coefficient, a Euclidean distance, a Mahalanobis distance, or a Tanimoto coefficient.

Pearson's correlation coefficient may identify whether corresponding samples are changed through similar linear transformation. Euclidean distance is a method of measuring the distance by extending the distance between two points on a plane into a multidimensional space, and Mahalanobis distance is a method of measuring the distance considering that one axis is severely compressed or expanded, not the same density of the coordinate axes in space. The Tanimoto coefficient is a value obtained by dividing the size of the intersection by the size of the union of two sets to check how similar the two sets are. When the degree of prognostic association of genes in two patient groups is compared using the Tanimoto coefficient, the set of genes with statistically significant values among mHi or mLi values is calculated. For example, when the prognostic association of each gene is calculated using the log-rank test in the mL patient group, a set A consisting of genes having a chi-square value of 1.64 or more, which can be said to be statistically significant, is obtained. In the same way, gene set B is obtained from the mH patient group. For these two sets, the value obtained by dividing the number of elements at the intersection of A and B by the number of elements at the union of A and B may be used as a value representing the similarity of the two sets.

The biomarker selection unit 400 may select a biomarker for dividing the patient group from a comparison value of the prognostic association value mHi of the high-expression patient group and the prognostic association value mLi of the low-expression patient group.

In order to select a biomarker, the biomarker selection unit 400 compares a prognostic association similarity value of a gene between two patient groups with a preset value.

For example, the biomarker selection unit 400 may determine whether to select a specific gene m as a biomarker depending on whether the similarity value calculated through the comparison of all genes in (mL1 and mH1), (mL2 and mH2),...(mLN and mHN) is greater than or equal to a preset value.

When the specific gene m is not selected as the biomarker, the biomarker selection unit 400 may instruct the patient group classification unit 100 to perform an operation on the next single gene again.

The system 1000 for discovering a novel target protein and a companion diagnostic biomarker therefor according to an embodiment of the present disclosure may further include a novel target protein selection unit 500.

The novel target protein selection unit 500 may select a novel target protein from the high-expression patient group or the low-expression patient group as the prognostic association value based on the biomarker.

For example, it may mean that the greater the prognostic association value, the worse the patient's prognosis is affected.

For example, in the case that in the low-expression patient group according to the expression level of a single gene m, the higher the expression of gene number 2, the shorter the death or metastasis time, and the prognostic association value that adversely affects the prognosis was the highest, and in the high-expression patient group according to the expression level of a single gene m, the higher the expression of gene number 34, the shorter the death or metastasis time, and the prognostic association value that adversely affects the prognosis was the highest, gene number 2 becomes a novel target protein for the low-expression patient group with low expression of a single gene m, and gene number 34 becomes a novel target protein for the high-expression patient group with high expression of a single gene m.

The biomarker for selecting patients in order to maximize the effect of the drug inhibiting the novel target protein searched as such is a single gene m.

As described above, the system 1000 for discovering a novel target protein and a companion diagnostic biomarker therefor according to an embodiment of the present disclosure may simultaneously discover an effective target protein in a patient group that shares generic features similar to a biomarker for predicting responsiveness to treatment.

New drug development companies are very interested in discovering biomarkers that select patient groups that can maximize the effectiveness of their drugs.

In the case of new drugs, the development period and development cost can be reduced depending on the presence or absence of a companion diagnostic biomarker.

Conventionally, a novel target protein cannot be discovered because the responsiveness to a therapeutic agent is identified after dividing the patient group who has already been prescribed a therapeutic agent with a known target by the presence or absence of a mutation in a specific protein. It is only possible to identify whether such a specific mutation will be a companion diagnostic biomarker for treatment.

However, the system 1000 for discovering a novel target protein and a companion diagnostic biomarker therefor according to an embodiment of the present disclosure may discover a novel target protein and a companion diagnostic biomarker therefor without prescription data for a drug inhibiting a protein that is expected to be a novel target.

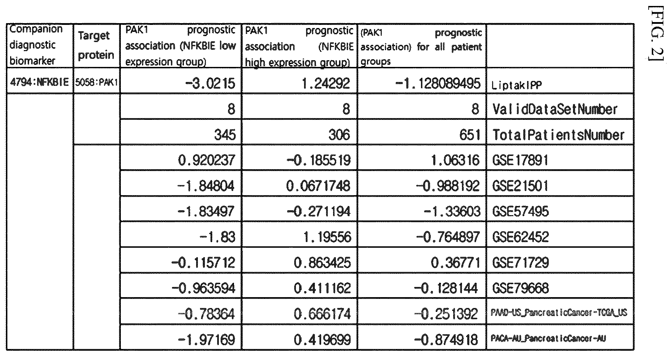

FIG. 2 is a diagram illustrating a portion of the actual results calculated using a system for discovering a novel target protein and a companion diagnostic biomarker therefor according to an embodiment of the present disclosure.

Referring to FIG. 2, the prognostic association value is calculated by a log-rank test based on iterative patient partitioning method. Negative values indicate that the gene expression level is high, and the patient's death or metastasis occurs quickly, resulting in a poor prognosis.

A total of 8 data sets were available, and data of a total of 651 patients were used.

Below is a brief description of each of the data sets used in the above calculations.
GSE17891: UK data published in April 2011, including a sample of 27 patients. Reference document: Collisson EA, Sadanandam A, Olson P, Gibb WJ et al. Subtypes of pancreatic ductal adenocarcinoma and their differing responses to therapy. Nat Med 2011 Apr;17(4):500-3
GSE21501: US data published in July 2010, including a sample of 130 patients. Reference document: Stratford JK, Bentrem DJ, Anderson JM, Fan C et al. A six-gene signature predicts survival of patients with localized pancreatic ductal adenocarcinoma. PLoS Med 2010 Jul 13;7(7):e1000307
GSE57495: US data published in August 2015, including a sample of 63 patients. Reference document: Chen DT, Davis-Yadley AH, Huang PY, Husain K et al. Prognostic Fifteen-Gene Signature for Early Stage Pancreatic Ductal Adenocarcinoma. PLoS One 2015;10(8):e0133562.
GSE62452: US data published in July 2016, including a sample of 130 patients. Reference document: Yang S, He P, Wang J, Schetter A et al. A Novel MIF Signaling Pathway Drives the Malignant Character of Pancreatic Cancer by Targeting NR3C2. Cancer Res 2016 Jul 1;76(13):3838-50.
GSE71729: US data published in September 2015, including a sample of 145 patients. Reference document: Moffitt RA, Marayati R, Flate EL, Volmar KE et al. Virtual microdissection identifies distinct tumor- and stroma-specific subtypes of pancreatic ductal adenocarcinoma. Nat Genet 2015 Oct;47(10):1168-78
GSE79668: US data published in June 2016, including a sample of 51 patients. Reference document: Kirby MK, Ramaker RC, Gertz J, Davis NS et al. RNA sequencing of pancreatic adenocarcinoma tumors yields novel expression patterns associated with long-term survival and reveals a role for ANGPTL4. Mol Oncol 2016 Oct;10(8):1169-82
PAAD-US: US data published to the International Cancer Research Consortium ICGC, including data on a total of 185 patients. Data address: https://dec.icge.org/releases/release_27/Projects/PAAD-US
PACA-AU: Australian data published to the International Cancer Research Consortium ICGC, including data on a total of 461 patients. Data address: https://dec.icge.org/releases/release_27/Projects/PACA-AU

The prognostic association value of PAK1 in each of the NFKBIE non-expressing group and the NFKBIE-expressing group was calculated by a log-rank test based on iterative patient partitioning method in each data set.

Upon reviewing the specific calculation method, for the data set GSE62452, when information on the period until death of each patient (days), death occurrence, NFKBIE expression, and PAK1 expression levels are given, the prognostic association score of PAK1 in the NFKBIE non-expressing group is calculated by the patient enumeration method, and the calculation method is as follows.

In GSE62452, as shown in Table 1 below, there are a total of 130 samples, of which 66 are cancerous tissues. Since the average expression level of NFKBIE in 66 cancerous tissues is 0.13574, only patients with an expression level of NFKBIE less than 0.13574 are selected.

**[Table 1]**

| **Sample ID** | **NFKBIE** | **PAK1** | **T.OS(Number of days until death)** | **E.OS (Death or not, 1=death, 0=stop tracking)** |
|---|---|---|---|---|
| GSM1527199 | 0.0924361 | 0.725367 | 27.375 | 1 |
| GSM1527139 | 0.0719572 | 0.697039 | 36.5 | 1 |
| GSM1527117 | 0.107975 | 0.83112 | 73 | 1 |
| GSM1527109 | 0.136983 | 0.943646 | 82.125 | 1 |
| GSM1527131 | 0.160193 | 0.779189 | 85.16666667 | 1 |
| GSM1527234 | 0.0648599 | 0.506486 | 97.33333333 | 1 |
| GSM1527183 | 0.198108 | 0.751111 | 127.75 | 1 |
| GSM1527191 | 0.0983756 | 0.756738 | 136.875 | 1 |
| GSM1527179 | 0.198062 | 0.927857 | 139.9166667 | 1 |
| GSM1527204 | 0.0834156 | 0.741575 | 161.2083333 | 1 |
| GSM1527200 | 0.169306 | 0.645679 | 179.4583333 | 1 |
| GSM1527198 | 0.182152 | 0.84668 | 191.625 | 1 |
| GSM1527175 | 0.2918 | 0.881733 | 194.6666667 | 1 |
| GSM1527155 | 0.112032 | 0.93257 | 206.8333333 | 1 |
| GSM1527107 | 0.149199 | 0.915945 | 209.875 | 1 |
| GSM1527163 | 0.217211 | 0.898843 | 234.2083333 | 1 |
| GSM1527171 | 0.170377 | 0.882882 | 270.7083333 | 1 |
| GSM1527223 | 0.116616 | 0.728521 | 282.875 | 1 |
| GSM1527196 | 0.0680492 | 0.738668 | 288.9583333 | 1 |
| GSM1527187 | 0.238311 | 0.934063 | 295.0416667 | 0 |
| GSM1527159 | 0.306631 | 0.906395 | 298.0833333 | 1 |
| GSM1527202 | 0.0998357 | 0.815202 | 298.0833333 | 1 |
| GSM1527185 | 0.0517133 | 0.505602 | 313.2916667 | 1 |
| GSM1527181 | 0.147486 | 0.920583 | 322.4166667 | 0 |
| GSM1527145 | 0.178356 | 0.900167 | 328.5 | 1 |
| GSM1527219 | 0.0715149 | 0.532571 | 331.5416667 | 1 |
| GSM1527133 | 0.103622 | 0.892387 | 352.8333333 | 1 |
| GSM1527125 | 0.115917 | 0.975128 | 383.25 | 1 |
| GSM1527193 | 0.107316 | 0.827322 | 392.375 | 1 |
| GSM1527141 | 0.218086 | 0.863629 | 401.5 | 1 |
| GSM1527218 | 0.0809268 | 0.764934 | 416.7083333 | 1 |
| GSM1527154 | 0.0623943 | 0.778882 | 419.75 | 1 |
| GSM1527207 | 0.0710075 | 0.619538 | 431.9166667 | 1 |
| GSM1527189 | 0.16918 | 0.91218 | 453.2083333 | 1 |
| GSM1527127 | 0.0836313 | 0.885285 | 486.6666667 | 1 |
| GSM1527177 | 0.142566 | 0.948263 | 498.8333333 | 0 |
| GSM1527173 | 0.16959 | 0.909593 | 526.2083333 | 0 |
| GSM1527123 | 0.198963 | 0.874086 | 593.125 | 1 |
| GSM1527225 | 0.0844458 | 0.676123 | 605.2916667 | 1 |
| GSM1527169 | 0.142231 | 0.914037 | 641.7916667 | 0 |
| GSM1527167 | 0.180534 | 0.877268 | 644.8333333 | 0 |
| GSM1527220 | 0.17064 | 0.883689 | 647.875 | 1 |
| GSM1527205 | 0.0867355 | 0.70674 | 653.9583333 | 1 |
| GSM1527165 | 0.160634 | 0.918809 | 663.0833333 | 0 |
| GSM1527213 | 0.15267 | 0.895445 | 666.125 | 1 |
| GSM1527210 | 0.178495 | 0.986706 | 696.5416667 | 1 |
| GSM1527143 | 0.180668 | 0.945885 | 705.6666667 | 1 |
| GSM1527161 | 0.174789 | 0.816583 | 717.8333333 | 0 |
| GSM1527135 | 0.235951 | 0.906498 | 751.2916667 | 1 |
| GSM1527151 | 0.192333 | 0.893421 | 839.5 | 0 |
| GSM1527149 | 0.195641 | 0.886273 | 842.5416667 | 1 |
| GSM1527157 | 0.138786 | 0.945639 | 857.75 | 0 |
| GSM1527147 | 0.168687 | 0.776631 | 882.0833333 | 1 |
| GSM1527209 | 0.0661931 | 0.624513 | 973.3333333 | 1 |
| GSM1527115 | 0.131654 | 0.907478 | 1091.958333 | 1 |
| GSM1527216 | 0.0616188 | 0.446232 | 1164.958333 | 0 |
| GSM1527137 | 0.0726089 | 0.676894 | 1216.666667 | 0 |
| GSM1527129 | 0.108316 | 0.902027 | 1244.041667 | 1 |
| GSM1527111 | 0.0866626 | 0.710018 | 1265.333333 | 1 |
| GSM1527215 | 0.0941973 | 0.573358 | 1277.5 | 0 |
| GSM1527212 | 0.0940205 | 0.567199 | 1396.125 | 1 |
| GSM1527232 | 0.128075 | 0.781619 | 1511.708333 | 1 |
| GSM1527105 | 0.144634 | 0.928392 | 1554.291667 | 1 |
| GSM1527230 | 0.075911 | 0.4696 | 2059.208333 | 0 |
| GSM1527228 | 0.100905 | 0.602254 | 2068.333333 | 0 |
| GSM1527227 | 0.0447046 | 0.338783 | 2153.5 | 0 |

With respect to the selected NFKBIE low-expression group patients, patients are sorted as shown in Table 2 according to the expression level of PAK1.

**[Table 2]**

| **Sample ID** | **NFKBIE** | **PAK1** | **T.OS (Number of days until death)** | **E.OS (Death or not, 1=death, 0=stop tracking)** |
|---|---|---|---|---|
| GSM1527227 | 0.0447046 | 0.338783 | 2153.5 | 0 |
| GSM1527216 | 0.0616188 | 0.446232 | 1164.958333 | 0 |
| GSM1527230 | 0.075911 | 0.4696 | 2059.208333 | 0 |
| GSM1527185 | 0.0517133 | 0.505602 | 313.2916667 | 1 |
| GSM1527234 | 0.0648599 | 0.506486 | 97.33333333 | 1 |
| GSM1527219 | 0.0715149 | 0.532571 | 331.5416667 | 1 |
| GSM1527212 | 0.0940205 | 0.567199 | 1396.125 | 1 |
| GSM1527215 | 0.0941973 | 0.573358 | 1277.5 | 0 |
| GSM1527228 | 0.100905 | 0.602254 | 2068.333333 | 0 |
| GSM1527207 | 0.0710075 | 0.619538 | 431.9166667 | 1 |
| GSM1527209 | 0.0661931 | 0.624513 | 973.3333333 | 1 |
| GSM1527225 | 0.0844458 | 0.676123 | 605.2916667 | 1 |
| GSM1527137 | 0.0726089 | 0.676894 | 1216.666667 | 0 |
| GSM1527139 | 0.0719572 | 0.697039 | 36.5 | 1 |
| GSM1527205 | 0.0867355 | 0.70674 | 653.9583333 | 1 |
| GSM1527111 | 0.0866626 | 0.710018 | 1265.333333 | 1 |
| GSM1527199 | 0.0924361 | 0.725367 | 27.375 | 1 |
| GSM1527223 | 0.116616 | 0.728521 | 282.875 | 1 |
| GSM1527196 | 0.0680492 | 0.738668 | 288.9583333 | 1 |
| GSM1527204 | 0.0834156 | 0.741575 | 161.2083333 | 1 |
| GSM1527191 | 0.0983756 | 0.756738 | 136.875 | 1 |
| GSM1527218 | 0.0809268 | 0.764934 | 416.7083333 | 1 |
| GSM1527154 | 0.0623943 | 0.778882 | 419.75 | 1 |
| GSM1527232 | 0.128075 | 0.781619 | 1511.708333 | 1 |
| GSM1527202 | 0.0998357 | 0.815202 | 298.0833333 | 1 |
| GSM1527193 | 0.107316 | 0.827322 | 392.375 | 1 |
| GSM1527117 | 0.107975 | 0.83112 | 73 | 1 |
| GSM1527127 | 0.0836313 | 0.885285 | 486.6666667 | 1 |
| GSM1527133 | 0.103622 | 0.892387 | 352.8333333 | 1 |
| GSM1527129 | 0.108316 | 0.902027 | 1244.041667 | 1 |
| GSM1527115 | 0.131654 | 0.907478 | 1091.958333 | 1 |
| GSM1527155 | 0.112032 | 0.93257 | 206.8333333 | 1 |
| GSM1527125 | 0.115917 | 0.975128 | 383.25 | 1 |

Then, when the patient with the lowest PAK1 expression (GSM1527227) is selected as the low PAK1 group and the remaining patients are selected as the high PAK1 group, a log-rank analysis results in a chi-square value of 2.106.

Next, except for the patient with the second expression rank of PAK1, only the remaining patients from the third rank patient were analyzed as the high PAK1 group for a log-rank, and a chi-square value of 2.186 was recorded.

In this manner, for all the i<j pairs satisfying i<j for the patient groups P(1),..., and P(n) arranged in the order of expression of PAK1, the average value of the n*(n-1)/2 chi-square values obtained by calculating the log-rank value for each case in which {P(1), ... , P(i)} is the PAK1 low-expression patient group and {P(j),... , P(n)} is the PAK1 high-expression patient group (Reference: Robust method for identification of prognostic gene signatures from gene expression profiles, Scientific Reportsvolume 7, Article number: 16926 (2017)).

Thereafter, the same operation was performed for the NFKBIE high expression group (the patient group with expression of 0.13574 or higher) to calculate the prognostic association score of PAK1 in the NFKBIE high expression group. This operation is performed for all available data sets.

Thereafter, z-values that can show the statistical significance from each data set were collected to calculate the final prognostic score, which is a single value. In the collection at this time, Liptak's z-value calculation method, which takes into account both the statistical value (z-value or p-value) and the number of patients used therefor (J Evol Biol. 2011 Aug;24(8):1836-41. doi: 10.1111/j.1420-9101.2011.02297.x. Epub 2011 May 23. Optimally weighted Z-test is a powerful method for combining probabilities in meta-analysis. Zaykin) was used. Liptak's z-value is known to follow the standard normal distribution, and therefore, the absolute value of the significant z-value at the significance level of 0.05 is approximately 1.64, and genes with greater z-values may be regarded as significant.

In the NFKBIE non-expressing group, it was identified that PAK1 has a poor prognosis in a number of data sets (<-1.64). The negative z value means that the higher the gene expression, the worse the patient's prognosis, and the positive z value means that the higher the gene expression, the better the patient's prognosis.

Accordingly, PAK1 may be a novel target protein in pancreatic cancer, and NFKBIE is a companion diagnostic biomarker for selecting patients who can expect the effect of a PAK1 inhibitor.

When the prognostic association score of PAK1 is calculated for all patients without classifying patients according to the expression of NFKBIE, it is a statistically insignificant value (-1.1280), which supports the concept that PAK1 can be found as a target protein only when patients are grouped based on the expression level of NFKBIE.

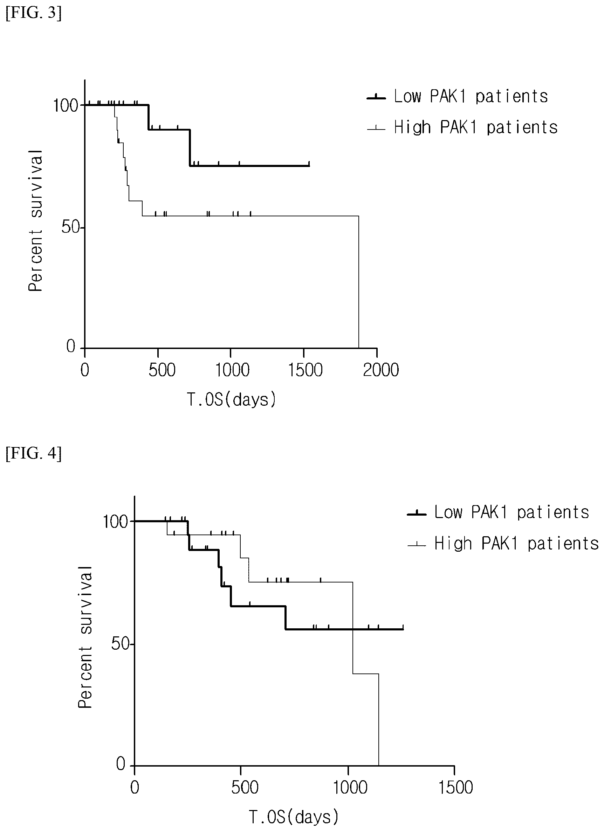

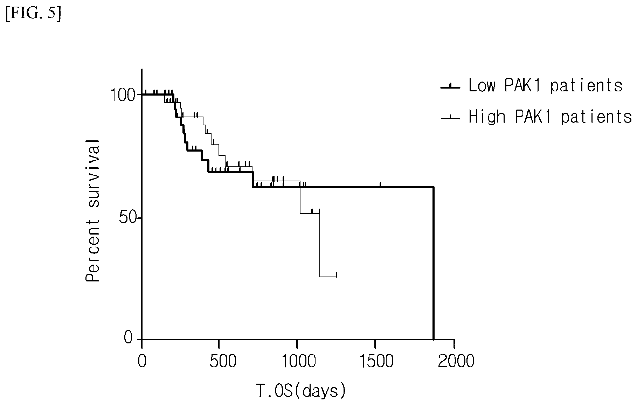

FIG. 3 is an experimental diagram illustrating the prognostic association values of PAK1 in a low-expression group of NFKBIE. FIG. 4 is an experimental diagram illustrating the prognostic association values of PAK1 in a high-expression group of NFKBIE. FIG. 5 is an experimental diagram illustrating the prognostic association values of PAK1 in all patients.

Referring to FIG. 3, it can be seen that the mortality rate of the patient group with high expression of PAK1 is higher than that of the patient group with low expression of PAK1 in the NFKBIE low expression group.

Referring to FIG. 4, it can be seen that the mortality rate of the patient group with high expression of PAK1 and the mortality rate of the patient group with low expression of PAK1 in the NFKBIE high expression group were not significantly different.

Referring to FIG. 5, it can be seen that there is no difference in the mortality rate according to the expression of PAK1 when all the patients are targeted without dividing the patient group according to the expression level of NFKBIE.

As such, PAK1 may be a novel target protein, and NFKBIE may be a companion diagnostic biomarker for selecting patients who can expect the effect of a PAK1 inhibitor.

Hereinafter, a method for discovering a novel target protein and a companion diagnostic biomarker therefor according to an embodiment of the present disclosure will be described. Hereinafter, for convenience of description, the reference numerals mentioned in FIG. 1 above will be mentioned and described, and the contents overlapping with those described earlier will be omitted.

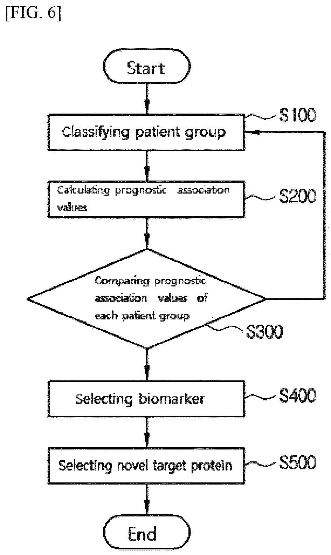

FIG. 6 is a flowchart of a method for discovering a novel target protein and a companion diagnostic biomarker therefor according to an embodiment of the present disclosure.

Referring to FIG. 6, a method for discovering a novel target protein and a companion diagnostic biomarker therefor according to an embodiment includes a first operation of classifying a patient group S 100, a second operation of calculating a prognostic association value S200, a third operation of comparing a prognostic association value of each patient group S300, a fourth operation of selecting a biomarker S400, and a fifth operation of selecting a novel target protein S500.

In the first operation S 100, the patient group classification unit 100 may specify a single gene, and a high-expression patient group and a low-expression patient group may be divided according to the expression level of the single gene.

In the second operation S200, the prognostic association calculation unit 200 may calculate prognostic association values of all genes in the high-expression patient group and the low-expression patient group.

In the third operation S300, the prognostic association comparison unit 300 may compare the prognostic association value of the high-expression patient group and the prognostic association value of the low-expression patient group for all genes.

When a specific single gene is not selected as a biomarker by comparing the prognostic association value of the high-expression patient group with the prognostic association value of the low-expression patient group, the patient group classification unit 100 performs an operation on the next single gene again.

In the fourth operation S400, the biomarker selection unit 400 may select a biomarker for dividing the patient group from the comparison of the prognostic association value of the high-expression patient group and the prognostic association value of the low-expression patient group.

In the fifth operation S500, the novel target protein selection unit may select a novel target protein as the prognostic association value in a high-expression patient group or a low-expression patient group based on the biomarker.

The present disclosure described above is not limited to the above-described embodiments and the accompanying drawings. It will be obvious to those having ordinary skill in the technical field to which the present disclosure pertains that various substitutions, modifications and changes are possible within the scope of the present disclosure.

## Claims

1. A system for discovering a target protein and a companion diagnostic biomarker therefor, the system comprising:
means for specifying a first gene and dividing a high-expression patient group and a low-expression patient group according to an expression level of the first gene;
means for calculating prognostic association values of all other genes in the high-expression patient group and the low-expression patient group according to the expression level of the first gene;
means for comparing prognostic association values of the high-expression patient group and prognostic association values of the low-expression patient group for all other genes, wherein a comparison value which represents the degree of similarity between the high-expression and low-expression patient groups is obtained;
means for selecting a biomarker to divide a patient group from the comparison value, wherein the biomarker is selected if the comparison value is greater than or equal to a preset value; and
means for selecting a target protein, wherein when a second gene has the highest prognostic association value that adversely affects the prognosis in the high-expression patient group or low-expression patient group according to the expression level of the first gene, then the second gene is selected as the target protein for that high-expression patient group or low-expression patient group according to the expression level of the first gene.

2. The system of claim 1, wherein the prognostic association value is calculated based on a gene expression level, disease recurrence period, and whether a disease has recurred or not, which are prognostic data of a patient.

3. The system of claim 1, wherein a reference expression value for dividing the high-expression patient group and the low-expression patient group is performed by an average value of a patient group or a step-miner algorithm.

4. The system of claim 1, wherein the prognostic association value is generated by any one of a log-rank test, a Cox hazard ratio, or a log-rank test based on iterative patient partitioning method.

5. The system of claim 1, wherein a comparison of the prognostic association value of the high-expression patient group and the prognostic association value of the low-expression patient group is performed by any one of a Pearson's correlation coefficient, an Euclidean distance, a Mahalanobis distance, or a Tanimoto coefficient.

6. A method for discovering a target protein and a companion diagnostic biomarker therefor, the method comprising:
specifying a first gene and dividing a high-expression patient group and a low-expression patient group according to an expression level of the first gene;
calculating prognostic association values of all other genes in the high-expression patient group and the low-expression patient group according to the expression level of the first gene, wherein a comparison value which represents the degree of similarity between the high-expression and low-expression patient groups is obtained;
comparing prognostic association values of the high-expression patient group and prognostic association values of the low-expression patient group for all other genes, wherein a comparison value which represents the degree of similarity between the high-expression and low-expression patient groups is obtained;
selecting a biomarker to divide a patient group from the comparison value, wherein the biomarker is selected if the comparison value is greater than or equal to a preset value; and
selecting a target protein, wherein when a second gene has the highest prognostic association value that adversely affects the prognosis in the high-expression patient group or low-expression patient group according to the expression level of the first gene, then the second gene is selected as the target protein for that high-expression patient group or low-expression patient group according to the expression level of the first gene.

7. The method of claim 6, wherein the prognostic association value is calculated based on a gene expression level, disease recurrence period, and whether a disease has recurred or not, which are prognostic data of a patient.

8. The method of claim 6, wherein a reference expression value for dividing the high-expression patient group and the low-expression patient group is performed by an average value of a patient group or a step-miner algorithm.

9. The method of claim 6, wherein the prognostic association value is generated by any one of a log-rank test, a Cox hazard ratio, or a log-rank test based on iterative patient partitioning method.

10. The method of claim 6, wherein a comparison of the prognostic association value of the high-expression patient group and the prognostic association value of the low-expression patient group is performed by any one of a Pearson's correlation coefficient, an Euclidean distance, a Mahalanobis distance, or a Tanimoto coefficient.

## Patentansprüche

1. System zum Auffinden eines Zielproteins und eines begleitenden diagnostischen Biomarkers dafür, wobei das System umfasst:
Mittel zum Spezifizieren eines ersten Gens und Unterteilen in eine Patientengruppe mit hoher Expression und eine Patientengruppe mit niedriger Expression entsprechend einem Expressionsniveau des ersten Gens;
Mittel zum Berechnen von prognostischen Assoziationswerten aller anderen Gene in der Patientengruppe mit hoher Expression und der Patientengruppe mit niedriger Expression entsprechend dem Expressionsniveau des ersten Gens;
Mittel zum Vergleichen von prognostischen Assoziationswerten der Patientengruppe mit hoher Expression und prognostischen Assoziationswerten der Patientengruppe mit niedriger Expression für alle anderen Gene, wobei ein Vergleichswert erhalten wird, welcher den Grad der Ähnlichkeit zwischen der Patientengruppe mit hoher Expression und der Patientengruppe mit niedriger Expression darstellt;
Mittel zum Auswählen eines Biomarkers zum Unterteilen einer Patientengruppe anhand des Vergleichswerts, wobei der Biomarker ausgewählt wird, wenn der Vergleichswert größer oder gleich einem voreingestellten Wert ist; und
Mittel zum Auswählen eines Zielproteins, wobei, für den Fall, dass ein zweites Gen den höchsten prognostischen Assoziationswert aufweist, der sich gemäß dem Expressionsniveau des ersten Gens nachteilig auf die Prognose in der Patientengruppe mit hoher Expression oder der Patientengruppe mit niedriger Expression auswirkt, das zweite Gen als Zielprotein für die Patientengruppe mit hoher Expression oder die Patientengruppe mit niedriger Expression gemäß dem Expressionsniveau des ersten Gens ausgewählt wird.

2. Das System nach Anspruch 1, wobei der prognostische Assoziationswert berechnet wird auf der Grundlage eines Genexpressionsniveaus, einer Krankheitsrezidivperiode und ob eine Krankheit rezidiviert ist oder nicht , wobei es sich um prognostische Daten eines Patienten handelt.

3. Das System nach Anspruch 1, wobei ein Referenz-Expressionswert zum Unterteilen der Patientengruppe mit hoher Expression und der Patientengruppe mit niedriger Expression durch einen Durchschnittswert einer Patientengruppe oder einen Step-Miner-Algorithmus bestimmt wird.

4. Das System nach Anspruch 1, wobei der prognostische Assoziationswert durch einen Log-Rank-Test, eine Cox-Hazard-Ratio oder einen Log-Rank-Test auf der Grundlage eines iterativen Patientenpartitionierungsverfahrens erzeugt wird.

5. Das System nach Anspruch 1, wobei ein Vergleich des prognostischen Assoziationswertes der Patientengruppe mit hoher Expression und des prognostischen Assoziationswertes der Patientengruppe mit niedriger Expression mittels eines Pearson-Korrelationskoeffizienten, eines euklidischen Abstands, eines Mahalanobis-Abstands oder eines Tanimoto-Koeffizienten durchgeführt wird.

6. Verfahren zum Auffinden eines Zielproteins und eines begleitenden diagnostischen Biomarkers dafür, wobei das Verfahren umfasst:
Spezifizieren eines ersten Gens und Unterteilen in eine Patientengruppe mit hoher Expression und eine Patientengruppe mit niedriger Expression entsprechend einem Expressionsniveau des ersten Gens;
Berechnen von prognostischen Assoziationswerten aller anderen Gene in der Patientengruppe mit hoher Expression und der Patientengruppe mit niedriger Expression entsprechend dem Expressionsniveau des ersten Gens, wobei ein Vergleichswert erhalten wird, welcher den Grad der Ähnlichkeit zwischen der Patientengruppe mit hoher Expression und der Patientengruppe mit niedriger Expression darstellt;
Vergleichen der prognostischen Assoziationswerte der Patientengruppe mit hoher Expression und der prognostischen Assoziationswerte der Patientengruppe mit niedriger Expression für alle anderen Gene, wobei ein Vergleichswert erhalten wird, welcher den Grad der Ähnlichkeit zwischen der Patientengruppe mit hoher Expression und der Patientengruppe mit niedriger Expression darstellt;
Auswählen eines Biomarkers zum Unterteilen einer Patientengruppe anhand des Vergleichswerts, wobei der Biomarker ausgewählt wird, wenn der Vergleichswert größer oder gleich einem voreingestellten Wert ist; und
Auswählen eines Zielproteins, wobei, für den Fall, dass ein zweites Gen den höchsten prognostischen Assoziationswert aufweist, der sich gemäß dem Expressionsniveau des ersten Gens negativ auf die Prognose in der Patientengruppe mit hoher Expression oder der Patientengruppe mit niedriger Expression auswirkt, das zweite Gen als Zielprotein für die Patientengruppe mit hoher Expression oder die Patientengruppe mit niedriger Expression gemäß dem Expressionsniveau des ersten Gens ausgewählt wird.

7. Verfahren nach Anspruch 6, wobei der prognostische Assoziationswert berechnet wird auf der Grundlage eines Genexpressionsniveaus, einer Krankheitsrezidivperiode und ob eine Krankheit rezidiviert ist oder nicht, , wobei es sich um prognostische Daten eines Patienten handelt.

8. Verfahren nach Anspruch 6, wobei ein Referenz-Expressionswert zum Unterteilen der Patientengruppe mit hoher Expression und der Patientengruppe mit niedriger Expression durch einen Durchschnittswert einer Patientengruppe oder einen Step-Miner-Algorithmus ermittelt wird.

9. Verfahren nach Anspruch 6, wobei der prognostische Assoziationswert durch einen Log-Rank-Test, eine Cox-Hazard-Ratio oder einen Log-Rank-Test auf der Grundlage eines iterativen Patientenpartitionierungsverfahrens erzeugt wird.

10. Verfahren nach Anspruch 6, wobei ein Vergleich des prognostischen Assoziationswerts der Patientengruppe mit hoher Expression und des prognostischen Assoziationswerts der Patientengruppe mit niedriger Expression mittels eines Pearson-Korrelationskoeffizienten, eines euklidischen Abstands, eines Mahalanobis-Abstands oder eines Tanimoto-Koeffizienten durchgeführt wird.

## Revendications

1. Système permettant d'identifier une protéine cible et un biomarqueur diagnostique associé à celle-ci, ledit système comprenant :
des moyens permettant de spécifier un premier gène et de subdiviser un groupe de patients en un groupe de patients à expression élevée et un groupe de patients à faible expression en fonction du niveau d'expression dudit premier gène;
des moyens pour calculer des valeurs d'association pronostiques de tous les autres gènes dans le groupe de patients à expression élevée et le groupe de patients à faible expression en fonction du niveau d'expression du premier gène;
des moyens pour comparer les valeurs d'association pronostique du groupe de patients à expression élevée et les valeurs d'association pronostique du groupe de patients à faible expression pour tous les autres gènes, obtenant ainsi une valeur comparative qui représente le degré de similitude entre le groupe de patients à expression élevée et le groupe de patients à faible expression;
des moyens pour sélectionner un biomarqueur afin de subdiviser un groupe de patients sur la base de la valeur comparative, le biomarqueur étant sélectionné lorsque la valeur comparative est supérieure ou égale à une valeur prédéfinie; et
des moyens pour sélectionner une protéine cible, dans lesquels, dans le cas où un deuxième gène présente la valeur d'association pronostique la plus élevée, qui a un effet défavorable sur le pronostic dans le groupe de patients à expression élevée ou le groupe de patients à faible expression en fonction du niveau d'expression du premier gène, le deuxième gène est sélectionné comme protéine cible pour le groupe de patients à expression élevée ou le groupe de patients à faible expression en fonction du niveau d'expression du premier gene.

2. Le système selon la revendication 1, dans lequel la valeur d'association pronostique est calculée sur la base d'un niveau d'expression génique, d'une période de récidive de la maladie et du fait qu'une maladie a récidivé ou non, qui sont des données pronostiques d'un patient.

3. Le système selon la revendication 1, dans lequel une valeur d'expression de référence pour diviser le groupe de patients à expression élevée et le groupe de patients à faible expression est déterminée par une valeur moyenne d'un groupe de patients ou un algorithme Step Miner.

4. Le système selon la revendication 1, dans lequel la valeur d'association pronostique est générée par un test du log-rank, un rapport de risque de Cox ou un test du log-rank basé sur un processus itératif de partitionnement des patients.

5. Le système selon la revendication 1, dans lequel une comparaison de la valeur d'association pronostique du groupe de patients à expression élevée et de la valeur d'association pronostique du groupe de patients à expression faible est effectuée à l'aide d'un coefficient de corrélation de Pearson, d'une distance euclidienne, d'une distance de Mahalanobis ou d'un coefficient de Tanimoto.

6. Procédé permettant d'identifier une protéine cible et un biomarqueur diagnostique associé à celle-ci, ledit procédé comprenant:
la spécification d'un premier gène et la subdivision en un groupe de patients à expression élevée et un groupe de patients à faible expression en fonction du niveau d'expression dudit premier gène;
le calcul des valeurs d'association pronostiques de tous les autres gènes dans le groupe de patients à expression élevée et le groupe de patients à faible expression en fonction du niveau d'expression du premier gène, obtenant ainsi une valeur comparative qui représente le degré de similitude entre le groupe de patients à expression élevée et le groupe de patients à faible expression;
comparer les valeurs d'association pronostiques du groupe de patients à expression élevée et les valeurs d'association pronostiques du groupe de patients à faible expression pour tous les autres gènes, obtenant ainsi une valeur comparative qui représente le degré de similitude entre le groupe de patients à expression élevée et le groupe de patients à faible expression;
sélectionner un biomarqueur pour subdiviser un groupe de patients sur la base de la valeur comparative, le biomarqueur étant sélectionné lorsque la valeur comparative est supérieure ou égale à une valeur prédéfinie; et
sélection d'une protéine cible, dans laquelle, dans le cas où un deuxième gène présente la valeur d'association pronostique la plus élevée, qui a un effet négatif sur le pronostic dans le groupe de patients à expression élevée ou dans le groupe de patients à faible expression en fonction du niveau d'expression du premier gène, le deuxième gène est sélectionné comme protéine cible pour le groupe de patients à expression élevée ou le groupe de patients à faible expression en fonction du niveau d'expression du premier gene.

7. Procédé selon la revendication 6, dans lequel la valeur d'association pronostique est calculée sur la base d'un niveau d'expression génique, d'une période de récidive de la maladie et du fait qu'une maladie a récidivé ou non, qui sont des données pronostiques d'un patient.

8. Procédé selon la revendication 6, dans lequel une valeur d'expression de référence pour diviser le groupe de patients à expression élevée et le groupe de patients à faible expression est déterminée par une valeur moyenne d'un groupe de patients ou un algorithme Step Miner.

9. Procédé selon la revendication 6, dans lequel la valeur d'association pronostique est générée par un test du log-rank, un rapport de risque de Cox ou un test du log-rank basé sur un procédé itératif de partitionnement des patients.

10. Procédé selon la revendication 6, dans lequel une comparaison de la valeur d'association pronostique du groupe de patients à expression élevée et de la valeur d'association pronostique du groupe de patients à faible expression est effectuée à l'aide d'un coefficient de corrélation de Pearson, d'une distance euclidienne, d'une distance de Mahalanobis ou d'un coefficient de Tanimoto.
